# FASCICULE DE BREVET EUROPEEN

(11) **EP 1 927 351 B1**
(45) Date de publication et mention de la délivrance du brevet: **06.10.2010**
(21) Numéro de dépôt: 07291392.4
(22) Date de dépôt: 23.11.2007
(51) Int. Cl.: A61K 31/165, A61P 25/00

(54) **Utilisation de l'agomélatine pour l'obtention de médicaments destinés au traitement de la leucomalacie périventriculaire**
Einsatz von Agomelatin zur Herstellung von Medikamenten zur Behandlung von periventrikulärer Leukomalazie
Use of agomelatine to obtain medication aimed at treating periventricular leukomalacia

(30) Priorité: 24.11.2006 FR 0610294
(43) Date de publication de la demande: 04.06.2008
(73) Titulaire: Les Laboratoires Servier, 92415 Courbevoie Cedex (FR)
(72) Inventeur: Spedding, Michael, 78110 Vesinet (FR); Mocaer, Elisabeth, 92200 Neuilly sur Seine (FR)

(56) Documents cités:
- EP-A- 1 564 202
- HUSSON ISABELLE ET AL: "Melatoninergic neuroprotection of the murine periventricular white matter against neonatal excitotoxic challenge" ANNALS OF NEUROLOGY, vol. 51, no. 1, janvier 2002 (2002-01), pages 82-92, XP002442543 ISSN: 0364-5134

## Description

La présente invention concerne l'utilisation de l'agomélatine ou *N*-[2-(7-méthoxy-1-naphtyl)éthyl]acétamide de formule (I) : 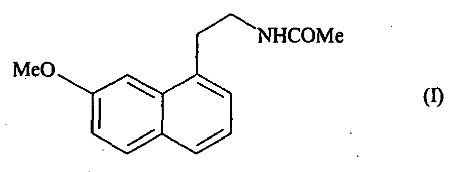 ainsi que ses hydrates, formes cristallines et sels d'addition à un acide ou à une base pharmaceutiquement acceptable, pour l'obtention de médicaments destinés au traitement de la leucomalacie périventriculaire.

L'agomélatine ou *N*-[2-(7-méthoxy-1-naphtyl)éthyl]acétamide présente la double particularité d'être d'une part agoniste sur les récepteurs du système mélatoninergique et d'autre part antagoniste du récepteur 5-HT_{2C}. Ces propriétés lui confèrent une activité dans le système nerveux central et plus particulièrement dans le traitement de la dépression majeure, des dépressions saisonnières, des troubles du sommeil, des pathologies cardiovasculaires, des pathologies du système digestif, des insomnies et fatigues dues aux décalages horaires, des troubles de l'appétit et de l'obésité.

L'agomélatine, sa préparation et son utilisation en thérapeutique ont été décrits dans les brevets européens EP 0 447 285 et EP 1 564 202.

La demanderesse a présentement découvert que l'agomélatine ou *N*-[2-(7-méthoxy-1-naphtyl)éthyl]acétamide, ainsi que ses hydrates, formes cristallines et sels d'addition à un acide ou à une base pharmaceutiquement acceptable, possédait des propriétés intéressantes permettant de l'utiliser dans le traitement de la leucomalacie périventriculaire.

La leucomalacie périventriculaire est la cause la plus fréquente de paralysie cérébrale chez l'enfant prématuré. Cette affection néonatale précoce est due à la formation de lésions uniques ou multiples de l'anneau de substance blanche périventriculaire survenant durant la vie prénatale ou néonatale, entre 20 et 34 semaines postconceptionnelles, exceptionnellement même jusqu'au terme. La leucomalacie périventriculaire est responsable de la majorité des séquelles motrices de la prématurité. Néanmoins, d'autres séquelles neurologiques ou complications, bien que moins fréquentes, peuvent être observées, qui témoignent de l'étendue des lésions au-delà de la substance blanche périventriculaire : anomalies de la croissance du périmètre crânien, témoignant essentiellement d'anomalies de la prolifération et/ou de la régression des dendrites ; déficit intellectuel, secondaire à une atteinte corticale associée ; troubles spécifiques du développement concernant un enfant sur deux ou trois à l'âge de l'entrée en cours préparatoire (i.e. 7 ans) ; déficits sensoriels, certes exceptionnels, secondaires à des atteintes des radiations auditives ou visuelles ; une fréquence accrue de la mort subite du nourrisson. A la douleur des parents confrontés au problème des séquelles neurologiques ou au décès de leur enfant s'ajoute le désarroi d'une équipe médicale totalement démunie face à l'apparition d'une leucomalacie périventriculaire étendue chez des nouveau-nés parfois indemnes de toute autre complication de la prématurité : en effet, aucune stratégie thérapeutique ne permet, de nos jours, de prévenir ou de limiter l'extension de telles lésions. Par ailleurs, l'augmentation de la fréquence des grossesses multiples ainsi que les limites toujours repoussées de la viabilité des grands prématurés ont pour conséquence une augmentation notoire de l'incidence de la leucomalacie périventriculaire, principal défi des néonatologistes. Récemment, les causes de la maladie ont été établies comme étant multifactorielles : des facteurs pré-conceptionnel, prénatal et périnatal peuvent être impliqués dans la survenue de lésions lors du développement du cerveau. Parmi ces facteurs, on citera les épisodes d'hypoxie-ischémie, les déséquilibres endocriniens, les facteurs génétiques, les désordres liés aux facteurs de croissance, les infections maternelles résultant en un excès de production de cytokines, l'exposition à des agents pro-inflammatoires... Ces multiples facteurs de risque ont des incidences moléculaires communes, en particulier un excès de libération d'aminoacides excitateurs ainsi qu'une production accrue d'espèces oxygénées réactives.
La demanderesse a présentement découvert que l'agomélatine possède un effet neuroprotecteur ayant pour effet de favoriser les mécanismes de réparation des lésions secondaires de la substance blanche périventriculaire. L'agomélatine constitue ainsi une nouvelle voie de traitement de la leucomalacie périventriculaire. De plus, l'agomélatine a la particularité d'être très bien toléré et de ne pas présenter de problèmes d'interactions médicamenteuses, ce qui en fait un traitement particulièrement bien adapté dans cette indication.

L'invention concerne donc l'utilisation de l'agomélatine, ainsi que ses hydrates, formes cristallines et sels d'addition à un acide ou à une base pharmaceutiquement acceptable, pour l'obtention de compositions pharmaceutiques destinées au traitement de la leucomalacie périventriculaire.

Particulièrement, l'invention concerne l'utilisation de l'agomélatine obtenue sous la forme cristalline II décrite dans la demande de brevet EP 1 564 202, pour l'obtention de compositions pharmaceutiques destinées au traitement de la leucomalacie périventriculaire.

Les compositions pharmaceutiques seront présentées sous des formes convenant aux administrations par voie orale, parentérale, transcutanée, nasale, rectale, perlinguale, et notamment sous forme de préparations injectables, comprimés, comprimés sublinguaux, glossettes, gélules, capsules, tablettes, suppositoires, crèmes, pommades, gels dermiques, etc...

Outre l'agomélatine, les compositions pharmaceutiques selon l'invention contiennent un ou plusieurs excipients ou véhicules choisis parmi des diluants, des lubrifiants, des liants, des agents de désintégration, des absorbants, des colorants, des édulcorants, etc...

A titre d'exemple et de manière non limitative, on peut citer :
◆ *pour les diluants :* le lactose, le dextrose, le sucrose, le mannitol, le sorbitol, la cellulose, la glycérine,
◆ *pour les lubrifiants :* la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

La posologie utile varie selon l'âge et le poids du patient, la voie d'administration, la nature de l'affection et des traitements éventuellement associés et s'échelonne entre 1 mg et 50 mg d'agomélatine par 24 heures.

De manière préférentielle, la dose journalière d'agomélatine sera de 25 mg par jour, avec possibilité d'augmenter à 50 mg par jour.

### Composition pharmaceutique :

Formule de préparation pour 1000 comprimés dosés à 25 mg :

| | |
|---|---|
| N-[2-(7-méthoxy-1-naphtyl)éthyl]acétamide | 25 g |
| Lactose monohydrate | 62 g |
| Stéarate de Magnésium | 1,3 g |
| Povidone | 9 g |
| Silice colloïdale anhydre | 0,3 g |
| Cellulose sodium glycolate | 30 g |
| Acide stéarique | 2,6 g |

### Etude pharmacologique

Les effets neuroprotecteurs de l'agomélatine ont été observés sur des bébés souris de 5 jours auxquels des lésions de la substance blanche du cerveau ont été induites par injection intracérébrale d'iboténate. Immédiatement après avoir reçu 10 µg d'iboténate, sont injectés par voie i.p. dans un volume final de 5 µl, 0,005 à 5 mg/kg d'agomélatine, 10 mg/kg de fluoxétine utilisé en tant qu'antidépresseur classique contrôle, ou du solvant seul (PBS : "Phosphate Buffered Saline"). Dans une seconde expérience, l'injection *i.p.* suivant l'injection intracérébrale d'iboténate se fait 2 heures, 4 heures ou 8 heures plus tard. Résultats : les souriceaux ayant reçu l'iboténate développent des lésions corticales ainsi que des lésions dans la substance blanche périventriculaire. La co-administration d'agomélatine se traduit par une réduction dose-dépendante des lésions de la substance blanche allant jusqu'à 59% de réduction pour une administration de 5 mg/kg. Aucun effet significatif sur les lésions de la substance blanche n'est observé lors de co-administration de fluoxétine ou de solvant seul.
La neuroprotection observée avec l'agomélatine décroît lorsque l'administration après l'injection d'iboténate est réalisée entre 0 et 4 heures puis une neuroprotection significative est restaurée lorsque l'administration est réalisée 8 heures après l'injection d'iboténate. Les résultats sont reportés ci-dessous :

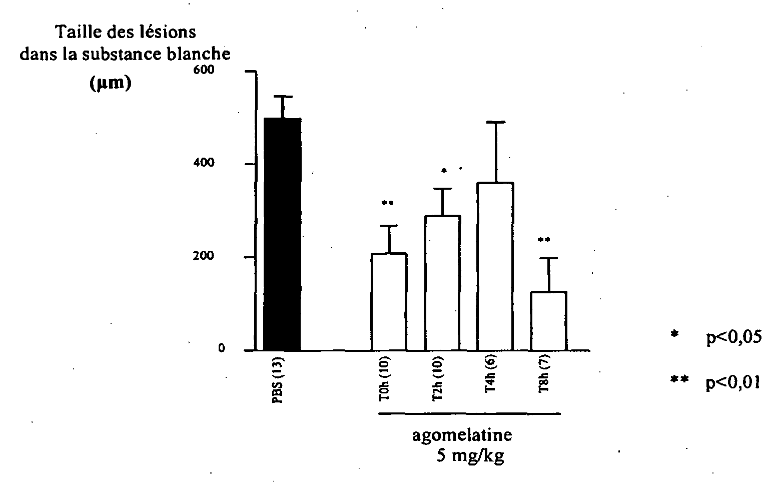

Les animaux qui ont reçu l'iboténate développent des lésions dont la taille augmente pendant les premières 24 heures suivant l'injection puis se stabilisent. Chez les animaux co-traités avec l'agomélatine (concomitamment ou après un délai de 8 heures), on observe les mêmes lésions pendant les premières 24 heures puis une régression très importante les 4 jours suivants.

## Revendications

1. Utilisation de l'agomélatine ou *N*-[2-(7-méthoxy-1-naphtyl)éthyl]acétamide ou un de ses hydrates, formes cristallines ainsi que ses sels d'addition à un acide ou à une base pharmaceutiquement acceptable, pour la fabrication d'un médicament destiné au traitement de la leucomalacie périventriculaire.

2. Utilisation selon la revendication 1 **caractérisée en ce que** l'agomélatine est obtenu sous la forme cristalline II.

3. Compositions pharmaceutiques contenant de l'agomélatine ou un de ses hydrates, formes cristallines ainsi que ses sels d'addition à un acide ou à une base pharmaceutiquement acceptable, seule ou en combinaison avec un ou plusieurs excipients pharmaceutiquement acceptables, pour l'utilisation dans le traitement de la leucomalacie périventriculaire.

4. Composition pharmaceutique selon la revendication 3 **caractérisée en ce que** l'agomélatine est obtenu sous la forme cristalline II.

5. Agomélatine ou N-[2-(7-méthoxy-1-naphtyl)éthyl]acétamide ou un de ses hydrates, formes cristallines ainsi que ses sels d'addition à un acide ou à une base pharmaceutiquement acceptable pour l'utilisation dans le traitement de la leucomalacie périventriculaire.

6. Forme cristalline II de l'agomélatine pour l'utilisation dans le traitement de la leucomalacie périventriculaire.

## Claims

1. Use of agomelatine, or *N*-[2-(7-methoxy-1-naphthyl)ethyl]acetamide, or one of its hydrates, crystalline forms, and also addition salts thereof with a pharmaceutically acceptable acid or base, in the manufacture of a medicament intended for the treatment of periventricular leukomalacia.

2. Use according to claim 1, **characterised in that** the agomelatine is obtained in crystalline form II.

3. Pharmaceutical compositions comprising agomelatine, or one of its hydrates, crystalline forms, and also addition salts thereof with a pharmaceutically acceptable acid or base, on its own or in combination with one or more pharmaceutically acceptable excipients, for use in the treatment of periventricular leukomalacia.

4. Pharmaceutical composition according to claim 3, **characterised in that** the agomelatine is obtained in crystalline form II.

5. Agomelatine, or *N*-[2-(7-methoxy-1-naphthyl)ethyl]acetamide, or one of its hydrates, crystalline forms, and also addition salts thereof with a pharmaceutically acceptable acid or base, for use in the treatment of periventricular leukomalacia.

6. Crystalline form II of agomelatine for use in the treatment of periventricular leukomalacia.

## Patentansprüche

1. Verwendung von Agomelatin oder *N*-[2-(7-Methoxy-1-naphthyl)-ethyl]-acetamid oder eines seiner Hydrate, Kristallformen sowie seiner Additionssalze mit einer pharmazeutisch annehmbaren Säure oder Base zur Herstellung eines Arzneimittels zur Behandlung von periventrikulärer Leukomalazie.

2. Verwendung nach Anspruch 1, **dadurch gekennzeichnet, dass** Agomelatin in der Kristallform II vorliegt.

3. Pharmazeutische Zubereitungen enthaltend Agomelatin oder eines seiner Hydrate, Kristallformen sowie seiner Additionssalze mit einer pharmazeutisch annehmbaren Säure oder Base, allein oder in Kombination mit einem oder mehreren pharmazeutisch annehmbaren Hilfsstoffen zur Verwendung bei der Behandlung von periventrikulärer Leukomalazie.

4. Pharmazeutische Zubereitung nach Anspruch 3, **dadurch gekennzeichnet, dass** Agomelatin in der Kristallform II vorliegt.

5. Agomelatin oder N-[2-(7-Methoxy-1-naphthyl)-ethyl]-acetamid oder eines seiner Hydrate, Kristallformen sowie seiner Additionssalze mit einer pharmazeutisch annehmbaren Säure oder Base zur Verwendung bei der Behandlung von periventrikulärer Laukomalazie.

6. Kristallform II von Agomelatin zur Verwendung bei der Behandlung der periventrikulären Leukomalazie.
